# EUROPEAN PATENT APPLICATION

(11) **EP 2 639 213 A1**
(43) Date of publication of application: **18.09.2013**
(21) Application number: 11839905.4
(22) Date of filing: 03.06.2011
(51) Int. Cl.: C07C 2/08, C07C 2/26, C07C 11/02, B01J 31/02

(54) **METHOD FOR PREPARING 1-OCTENE BY OLIGOMERIZATION OF ETHYLENE**

(30) Priority: 11.11.2010 CN 201010543099
(71) Applicant: Petrochina Company Limited, Beijing 100724 (CN)
(72) Inventor: WANG, Gang, Beijing 100724 (CN); ZHANG, Baojun, Beijing 100007 (CN); LI, Jianzhong, Beijing 100007 (CN); WANG, Sihan, Beijing 100007 (CN); QU, Jiabo, Beijing 100007 (CN); HE, Defu, Beijing 100007 (CN); WANG, Guizhi, Beijing 100007 (CN); CHEN, Qian, Beijing 100007 (CN); YU, Buwei, Beijing 100007 (CN); ZHANG, Deshun, Beijing 100007 (CN); WANG, Libo, Beijing 100007 (CN); WANG, Yali, Beijing 100007 (CN); LIANG, Liwei, Beijing 100007 (CN); GAO, Yuxin, Beijing 100007 (CN); LI, Jie, Beijing 100007 (CN); ZHANG, Wenchao, Beijing 100007 (CN); LI, Hua, Beijing 100007 (CN); GAO, Xiaoyu, Beijing 100007 (CN); ZHA, Shouhui, Beijing 100007 (CN); HUANG, Fuling, Beijing 100007 (CN); WEI, Jing, Beijing 100007 (CN); WANG, Xiuhui, Beijing 100007 (CN); YU, Xiangmin, Beijing 100007 (CN); ZHAO, Jingying, Beijing 100007 (CN); HAN, Xuemei, Beijing 100007 (CN); SONG, Chunfeng, Beijing 100007 (CN); WEI, Junfeng, Beijing 100007 (CN); MENG, Rui, Beijing 100007 (CN)
(74) Representative: Regimbeau
(86) International application number: PCT/CN2011/000945
(87) International publication number: WO 2012/062028

(57) **Abstract**

Disclosed is a method for preparing 1-octene in the presence of a catalyst system for ethylene oligomerization, which includes: premixing a part of ethylene gas as raw material with a solvent, mixing with the components a+b, c and d of the catalyst system, and then sending in a reactor; directly sending the rest of ethylene gas in the reactor; discharging the resultant liquid from the upper part of the reactor into an overflow channel; adding a catalysis stopping agent to the overflow channel; and then separating the resultant liquid. The advantages of the method are high selectivity of 1-octene and high catalytic activity.

## Description

### BACKGROUND

### Technical Field

The present invention relates to a method for preparing 1-octene in the presence of a catalyst system for ethylene oligomerization.

### Related Art

EP 0608447A1 reports a chromium-based catalyst composition which is used as a catalyst for ethylene oligomerization and/or copolymerization, wherein a chromium containing compound is used as a first component of the catalyst composition; a pyrrole compound is used as a second component of the chromium-based catalyst composition; and a Lewis acid activator and/or a metal alkylate activator is used as a third component of the catalyst component. Further, in EP 0608447A1, it is also pointed out that an optional halogen source may be further used as a fourth component of the catalyst composition, wherein the halogen source may be inorganic halides, or be various types of organic halides.

In JP 0832519, a Sn(OSO₂F₃)₂ compound is used to replace the halogen source as the fourth component in EP 0608447A1, to form a new quaternary chromium-based catalyst composition. However, the activity and selectivity of the quaternary chromium-based catalyst are not significantly improved.

USP 5,910,619 reports use of 1,2,3,4,5,6-hexachlorocyclohexane as a modifier, to form a quaternary catalyst composition. Although the activity of the catalyst is relatively improved, the requirement still cannot be met. The performance of the catalyst is expected to be further improved, so as to improve the catalytic activity.

The above ethylene oligomerization methods focus on the research of the catalyst. Although in WO 99/19380, the ethylene oligomerization method is studied, similar to the situations in other patents, the reaction is substantially carried out in an ordinary autoclave, which causes the problem of non-uniform mass transfer and heat transfer. As the reaction is a highly exothermic reaction, and the heat release rate is quick in an initial reaction phase, an excessively high local temperature is generally caused due to a slow heat transfer rate, leading to inactivation of a part of the catalyst, which affects the efficiency of the catalyst. Furthermore, due to a slow mass transfer rate in such autoclave, the catalytic performance of the complex catalyst is influenced, so that the catalytic activity is lowered, and the reaction time is generally long, resulting in increased energy consumption.

### SUMMARY

The present invention is directed to a method for preparing 1-octene in the presence of a catalyst system for ethylene oligomerization, so that a 1-octene product is prepared in a short reaction time, the activity of the catalyst is high, the selectivity of 1-octene is high, and the energy consumption of the whole method is low.

According to the method for preparing 1-octene in the presence of a catalyst system for ethylene oligomerization, before the raw material ethylene is fed to the reactor, a part of the raw material ethylene is premixed with a solvent in a premixer and/or a pipe, for the purpose of sufficient mass transfer between ethylene and the solvent, to increase the solubility of ethylene in the solvent to as high as 20%, thereby improving the yield of the product, and improving the single pass conversion rate of ethylene. The premixed solution of ethylene is then mixed with solutions of components a+b, c and d of the catalyst respectively, and fed to the reactor together with the rest of the raw material ethylene for reaction. The resultant liquid is fed to a separator for separating the product, in which 1-octene is directly used as a comonomer for LLDPE, the solvent is recycled, and the remaining catalyst is recovered. Before being fed to the separator, the resultant liquid is sampled for analysis.

### Gas distributor

The gas distributor is a stainless steel pipe having some pores opened at the middle part, and the diameter of the pores is 0.2-90%, generally 5-70%, and preferably 20-50% of the diameter of the stainless steel pipe.

### Catalyst and solvent

For the catalyst and the solvent used in preparation of 1-octene through ethylene oligomerization, see Chinese Patent entitled "Catalyst Composition for Ethylene Oligomerization and Use thereof" (Application No.: 200610057254.2).

The catalyst includes a chromium compound **a**, a ligand **b** containing P and N, an activator c, and a promoter **d**, wherein
**a** is chromium acetylacetonate, chromium chloride tetrahydrofuran and/or chromium isooctanoate;
**b** has a general formula below: 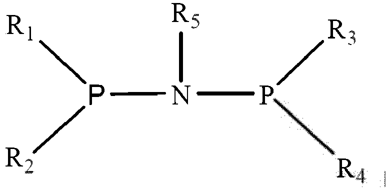 wherein R₁, R₂, R₃, and R₄ are phenyl, benzyl, fluorenyl or naphthyl; and R₅ is isopropyl, butyl, cyclopropyl, cyclopentyl, cyclohexyl or fluorenyl;
**c** is methylaluminoxane, ethylaluminoxane, propylaluminoxane and/or butylaluminoxane;
**d** has a general formula of X₁R₆X₂, wherein X₁ and X₂ are F, Cl, Br, I or an alkoxy; and R₆ is an alkyl or an aryl; and
the molar ratio of a, b, c and d is 1:0.5-10:50-3000:0.5-10.
Reaction conditions

For the reaction pressure and the reaction temperature in the reactor, see Chinese Patent entitled "Catalyst Composition for Ethylene Oligomerization and Use thereof" (Application No.: 200610057254.2).

The reaction temperature is 30-200°C; the reaction pressure is 0.5-20.0 MPa; and the time is 0.1-2 hours.

The inlet amount of the catalyst and the solvent satisfies the condition that the concentration of the component a of the catalyst in the reactor is 1-10 ppm, and desirably 1-3 ppm.

The ratio of the weight flow of ethylene fed into the premixer to the total weight flow of ethylene fed into the reactor may be any value that can satisfy the reaction condition, and is generally 0-100%, desirably 20-90%, and preferably 60-80%.

### BRIEF DESCRIPTION OF THE DRAWINGS

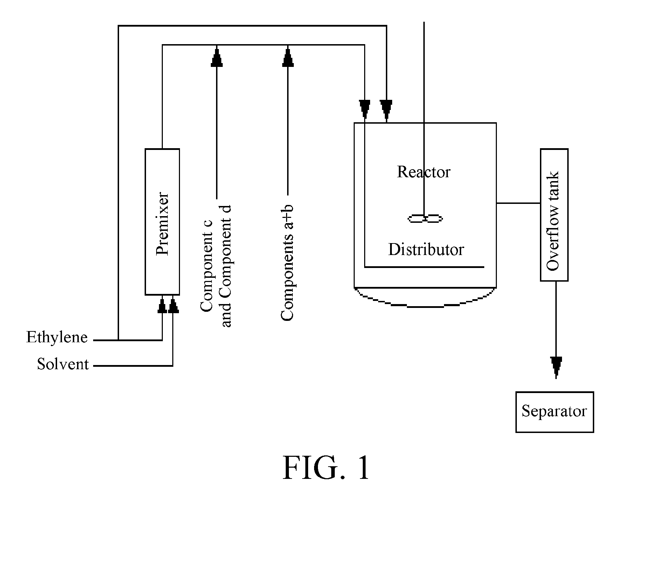

FIG. 1 is a simplified flow chart of preparing 1-octene through ethylene oligomerization.

### DETAILED DESCRIPTION

According to the method for preparing 1-octene through ethylene oligomerization of the present invention, before the raw material ethylene is fed to a reactor, a part of the raw material ethylene is premixed with a solvent in a premixer and/or a pipe, the premixed solution of ethylene is mixed with solutions of components a+b, c and d of a catalyst respectively, and then fed to the reactor via pores on a gas distribution pipe located at the bottom of the reactor; the rest of the raw material ethylene is directly fed to the reactor from the top of the reactor for reaction; the reactor is washed with cyclohexane, dried for 1 hour at 100°C in vacuum, cooled to room temperature, and replaced with ethylene, an ethylene inlet valve is opened, the pressure in the reactor is kept at 5.0 Mpa, the solvent together with the components a+b, c and d are introduced at a certain flow rate, the temperature in the reactor is kept at 50°C, an outlet valve of an overflow tank is opened, the resultant liquid is fed to a separator for separating the product, in which 1-octene is directly used as a comonomer for LLDPE, the solvent is recycled, and the remaining catalyst is recovered. Before being fed to the separator, the resultant liquid is sampled for analysis.

The premixer may be one or more stainless steel pipes having a length to diameter ratio of greater than 20 or a stainless steel autoclave, in which a filler may be filled.

The gas distributor is a stainless steel pipe having some pores opened at the middle part, and the diameter of the pores is 0.2-90%, generally 5-70%, and preferably 20-50% of the diameter of the stainless steel pipe.

### Catalyst and solvent

For the catalyst and the solvent used in preparation of 1-octene through ethylene oligomerization, see Chinese Patent entitled "Catalyst Composition for Ethylene Oligomerization and Use thereof" (Application No.: 200610057254.2).

### Reaction conditions

For the reaction pressure and the reaction temperature in the reactor, see Chinese Patent entitled "Catalyst Composition for Ethylene Oligomerization and Use thereof" (Application No.: 200610057254.2).

The inlet amount of the catalyst and the solvent satisfies the condition that the concentration of the component a of the catalyst in the reactor is 1-10 ppm, and desirably 1-3 ppm.

The ratio of the weight flow of ethylene fed into the premixer to the total weight flow of ethylene fed into the reactor may be any value that can satisfy the reaction condition, and is generally 0-100%, desirably 20-90%, and preferably 60-80%.

**Table 1 Process conditions and results**

| Example No. | Average residence time | Percentage of the diameter of the pore on the distributor to the diameter of the stainless steel pipe | Percentage of ethylene fed into the premixer to the total amount of ethylene | Catalytic activity (g product /gCr·h) | Selectivity of 1-ocetene (%) |
|---|---|---|---|---|---|
| 1 | 0.5 | 0.2 | 80 | 906836 | 70.1 |
| 2 | 0.5 | 10 | 80 | 1017396 | 71.2 |
| 3 | 0.5 | 30 | 80 | 1168633 | 73.1 |
| 4 | 0.5 | 60 | 80 | 1153468 | 72.6 |
| 5 | 0.5 | 80 | 80 | 1114923 | 72.0 |
| 6 | 0.5 | 90 | 80 | 986284 | 71.8 |
| 7 | 0.2 | 30 | 80 | 1106664 | 72.6 |
| 8 | 0.5 | 30 | 80 | 1168633 | 73.0 |
| 9 | 1 | 30 | 80 | 1030970 | 72.8 |
| 10 | 1.5 | 30 | 80 | 908903 | 71.6 |
| 11 | 2 | 30 | 80 | 863907 | 71.3 |
| 12 | 0.5 | 30 | 0 | 939604 | 70.2 |
| 13 | 0.5 | 30 | 20 | 959845 | 72.1 |
| 14 | 0.5 | 30 | 60 | 1029584 | 72.5 |
| 15 | 0.5 | 30 | 80 | 1168633 | 73.2 |
| 16 | 0.5 | 30 | 90 | 1101934 | 69.6 |

Other reaction conditions:
reaction temperature: 50°C, reaction pressure: 5.0 Mpa, catalyst formulation ratio a:b:c:d=1:1.05:300:5, modifier: 1,1,2,2-tetrachloroethane, solvent: cyclohexane, total inlet amount of ethylene: 20 kg/h, total inlet amount of solvent: 13 kg/h.

### Industrial Applicability

The process is applicable to preparation of 1-octene through ethylene oligomerization, and has a high catalytic activity and a high selectivity of 1-octene.
a. In the method, the reactants are mixed uniformly, with good effect of mass transfer and heat transfer.
b. High selectivity of 1-octene of greater than 70%.
c. High catalytic activity of the catalyst of greater than 1 x 10⁶ g product/gCr·h.

## Claims

1. A method for preparing 1-octene through ethylene oligomerization, **characterized in that**:
(1) premixing a part of the ethylene gas as raw material with a solvent in a premixer, then mixing with components a+b, c and d of a catalyst system, and feeding to a reactor via a gas distributor, and directly feeding the rest of the ethylene gas as raw material to the reactor;
wherein the catalyst comprises a chromium compound **a**, a ligand **b** containing P and N, an activator **c**, and a promoter **d**;
wherein **a** is chromium acetylacetonate, chromium chloride tetrahydrofuran and/or chromium isooctanoate;
**b** has a general formula below: 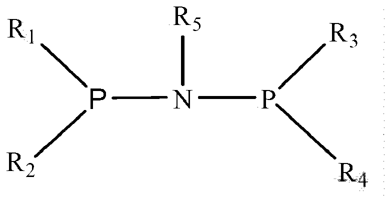 wherein R₁, R₂, R₃, and R₄ are phenyl, benzyl, fluorenyl or naphthyl; and R₅ is isopropyl, butyl, cyclopropyl, cyclopentyl, cyclohexyl or fluorenyl;
**c** is methylaluminoxane, ethylaluminoxane, propylaluminoxane and/or butylaluminoxane;
**d** has a general formula of X₁R₆X₂, wherein X₁, and X₂ are F, Cl, Br, I or an alkoxy;
and R₆ is an alkyl or an aryl; and
the molar ratio of a, b, c and d is 1:0.5-10:50-3000:0.5-10; and
(2) discharging the resultant liquid from the upper part of the reactor to an overflow tank, adding a catalysis stopping agent to the overflow tank, and then feeding the reaction product to a separator.

2. The method for preparing 1-octene through ethylene oligomerization according to claim 1, **characterized in that**, the premixer is one or more stainless steel pipes having a length to diameter ratio of greater than 20 or a stainless steel autoclave, in which a filler may be filled.

3. The method for preparing 1-octene through ethylene oligomerization according to claim 1, **characterized in that**, the gas distributor is a stainless steel pipe having some pores opened at the middle part, and the diameter of the pores is 0.2%-90% of the diameter of the stainless steel pipe.

4. The method for preparing 1-octene through ethylene oligomerization according to claim 3, **characterized in that**, the diameter of the pores on the gas distributor is 5%-70% of the diameter of the stainless steel pipe.

5. The method for preparing 1-octene through ethylene oligomerization according to claim 1, **characterized in that**, the ratio of the weight flow of ethylene fed into the premixer to the total weight flow of ethylene fed into the reactor is 20-90%.

6. The method for preparing 1-octene through ethylene oligomerization according to claim 1, **characterized in that**, the inlet amount of the catalyst and the solvent satisfies the condition that the concentration of the component a of the catalyst in the reactor is 1-10 ppm.
